Europäisches Patentamt

European Patent Office

Office européen des brevets

(11) Veröffentlichungsnummer: **0 425 673 A1**

(12) **EUROPÄISCHE PATENTANMELDUNG**
veröffentlicht nach Art. 158 Abs. 3
EPÜ

(21) Anmeldenummer: 89909906.3

(22) Anmeldetag: 28.04.89

(86) Internationale Anmeldenummer:
PCT/SU89/00113

(87) Internationale Veröffentlichungsnummer:
WO 90/10472 (20.09.90 90/22)

(51) Int. Cl.⁵: **A61N 1/36**

(30) Priorität: 06.03.89 SU 129589

(43) Veröffentlichungstag der Anmeldung:
08.05.91 Patentblatt 91/19

(84) Benannte Vertragsstaaten:
AT DE FR GB IT NL SE

(71) Anmelder: OSOBOE KONSTRUKTORSKOE
BJURO "RITM" PRI TAGANROGSKOM
RADIOTEKHNICHESKOM INSTITUTE IMENI
V.D.KALMYKOVA
ul. Lenina 99
Taganrog,347900(SU)

(72) Erfinder: KARASEV, Alexandr Alexandrovich
Mariupolskoe shosse, 27/1-164
Taganrog, 347939(SU)
Erfinder: ZAKHAREVICH, Vladislav
Georgievich
ul. P. Tolyatti, 22/3-37
Taganrog, 347919(SU)
Erfinder: REVENKO, Alexandr Nikolaevich
Mariupolskoe shosse, 27/1-90
Taganrog, 347939(SU)
Erfinder: KIBIREV, Alexandr Alexandrovich
ul. Vodoprovodnaya, 19-47
Taganrog, 347902(SU)
Erfinder: DYGAI, Alexandr Ivanovich
ul. B. Bulvarnaya, 10/1-18
Taganrog, 347900(SU)
Erfinder: NECHUSHKIN, Alexandr Ivanovich
ul. B. Cherkizovskaya, 6-6-57
Moscow, 107061(SU)

(74) Vertreter: Lehn, Werner, Dipl.-Ing. et al
Hoffmann, Eitle & Partner Patentanwälte
Arabellastrasse 4
W-8000 München 81(DE)

(54) **VERFAHREN ZUR ERZEUGUNG VON FÜR DIE ANREGUNG BIOLOGISCHER OBJEKTE BESTIMMTEN ELEKTRISCHEN IMPULSEN UND EINRICHTUNG ZU SEINER DURCHFÜHRUNG.**

(57) Das Verfahren besteht darin, daß man Impulse mit einer vorgegebenen Frequenz erzeugt, jeden Impuls längenmoduliert, Serien von Anregungsimpulsen mit einer vorgegebenen Seriendauer und einer vorgegebenen Pausenzeit zwischen ihnen formiert, die eine Serie bildenden Anregungsimpulse verstärkt, die Dauer der dem Objekt zuzuführenden Serie entsprechend einer Auswertung elektrophysikalischer Zustandsparameter des Zwischenelektrodenbereiches des biologischen Objektes verändert, die gleichzeitig mit der Anregung vorgenommen wird. Die Einrichtung enthält einen Impulsgenerator (6), einen Pulslängenmodulator (9), einen Anregungsimpuls-Leistungsverstärker (10), einen ersten Former (19) eines die Reaktion des Objektes auf den Anregungsimpuls bestimmenden Signals, einen Impulsgenerator (22) für Impulse einer veränderbaren Dauer, die die Dauer der Anregungsimpulsserie festlegt, einen zweiten Former (25) eines Signals, das eine Dauer hat, die der Seriendauer entspricht, und einen Höchstpegelbegrenzer (26) für das Modulationssteuerungssignal.

FIG. 2

## VERFAHREN ZUR ERZEUGUNG VON FÜR DIE ANREGUNG BIOLOGISCHER OBJEKTE BESTIMMTEN ELEKTRISCHEN IMPULSEN UND EINRICHTUNG ZU SEINER DURCHFÜHRUNG

Gebiet der Technik

Die Erfindung betrifft elektrotherapeutische Apparate und bezieht sich insbesondere auf Verfahren zur Erzeugung von für die Anregung biologischer Objekte bestimmten elektrischen Impulsen und auf eine Einrichtung zu deren Durchführung.

Stand der Technik

Bekannt ist ein Verfahren zur Anregung eines biologischen Objektes, bei dem man elektrische Impulse zu dessen Anregung erzeugt.

Das Verfahren besteht in einer nachstehend angegebenen Reihenfolge von Verfahrensschritten: man führt eine Anregung mit einem Referenzreiz, dann eine Indikationsanregung zur Bestimmung der Reaktion des Objektes und eine Heilanregung durch, wozu man elektrische Impulse mit vorgegebener Frequenz und Dauer formiert. Man verstärkt diese Impulse und führt sie zur Anregung innerhalb einer gewissen Zeitspanne zu. Dann nimmt man wieder die Indikationsanregung vor und urteilt darüber, ob die Heilanregung abgebrochen oder fortgesetzt werden muß (US,A,4505275).

Die nach diesem Verfahren erzeugten elektrischen Impulse weisen unveränderliche Parameter innerhalb der Periode der Heilanregung auf, sie lassen sich nicht einem laufenden Zustand des Anregungsobjektes anpassen. Eine mehrfache Heilanregung verlängert die Einwirkungsprozedur, wodurch die Wahrscheinlichkeit einer Überdosierung erhöht wird. All dies führt zu einer Verminderung des therapeutischen Effektes.

Bekannt ist ein Verfahren zur Erzeugung von elektrischen Impulsen für die Anregung neuromuskulärer Strukturen des Stütz- und Bewegungsapparates und der Organe, welches darin besteht, daß man Impulse mit einer vorgegebehen Frequenz erzeugt, jeden Impuls in der Länge moduliert, eine Serie von Impulsen mit einer vorgegebenen Seriendauer und einer vorgegebenen Pausenzeit zwischen ihnen unter linearer Verlängerung und Verkürzung der Dauer der Impulse vorgegebener Frequenz formiert, die eine Serie bildenden Anregungsimpulse verstärkt und diese dem biologischen Objekt zuführt, wobei man eine maximale Dauer der Impulse vorgegebener Frequenz in der Serie entsprechend der individuellen Empfindlichkeit des biologischen Objektes (SU,A,1169669) einstellt.

Die Parameter der einwirkenden Impulsserien werden versuchsweise bei maximaler Antwort des Objektes unter physiologisch komfortablen Bedingungen Bedingungen eingestellt. Dabei ist keine individuelle Dosierung vorgesehen, was eine Überdosierung hervorrufen kann. Dis Impulse werden einem laufenden Zustand des Objektes nicht angepaßt, und all dies setzt den gesamten therapeutischen Effekt herab.

Es ist eine Einrichtung zur Erzeugung von elektrischen Anregungsimpulsen bekannt, die einen Impulsgenerator, einen mit diesem verbundenen Modulator, einen Verstärker und eine Ausgangsstufe (SU,A,1069832) enthält.

Eine Impulsspannung rechteckiger Form wird vom Impulsgenerator auf einen Eingang des Modulators gegeben. Seinem zweiten Eingang werden Impulse von einem Modulationsfrequenzmodulator zugeführt. Am Ausgang des Modulators entsteht eine impulsmodulierte Trägerfrequenzspannung rechteckiger Form, die zum Eingang des Verstärkers gelangt und die nach der Verstärkung vom Ausgang des letzteren auf das biologische Objekt gegeben wird.

Bei dieser Einrichtung werden die Impulse mit starr stabilisierten, im voraus eingestellten Parametern erzeugt. Bei dieser Einrichtung ist keine Möglichkeit gegeben, den Zustand des biologischen Objektes und die Dynamik der Änderung dieses Zustandes auszuwerten, was zu einer ungerechtfertigten Verschleppung der Anregungsprozedur und zu einer eventuellen Überdosierung führt.

Bekannt ist auch ein elektrischer Anreger, der -in Reihe geschaltet- einen Impulsgenerator, einen Dauerformer, einen Amplitudenformer in Form eines Leistungsverstärkers und eine aktive Elektrode enthält, die an einen Gewebeabschnitt (SU,A, 1011130) angelegt wird.

Der Generator liefert Impulse mit gewünschter Anregungsperiode, von denen jeder Impuls den Reizdauerformer auslöst, der den Amplitudenformer steuert. Zugleich erzeugt der letztere einen Stromimpuls, der über die Elektroden fließt.

Eine solche Einrichtung besitzt dieselben Nachteile, die der früher betrachteten bekannten Einrichtung eigen sind.

Offenbarung der Erfindung

Der Erfindung liegt die Aufgabe zugrunde, ein Verfahren zur Erzeugung von für die Anregung von biologischen Objekten bestimmten elektrischen Impulsen und eine Einrichtung zu dessen Durchführung zu entwickeln, welche es gestatten, die elektrischen Impulse durch Änderung des Auswertungsparameters des Zustandes des biologischen Objektes zu verändern und damit diese Impulse an den Zustand des anzuregenden biologischen Objekt anzupassen.

Diese Aufgabe wird dadurch gelöst, daß man im Verfahren zur Erzeugung von für die Anregung biologischer Objekte bestimmten elektrischen Impulsen, bei dem man Impulse mit einer vorgegebenen Frequenz erzeugt, jeden Impuls längenmoduliert, Serien von Anregungsimpulsen mit einer vorgegebenen Seriendauer und einer vorgegebenen Pausenzeit zwischen ihnen unter linearer Verlängerung und Verkürzung der Dauer der Impulse vorgegebener Frequenz formiert, die eine Serie bildenden Anregungsimpulse verstärkt und diese über Elektroden einem biologischen Objekt zuführt, wobei man die maximale Dauer der Impulse vorgegebener Frequenz in der Serie entsprechend der individuellen Empfindlichkeit des biologischen Objektes einstellt, erfindungsgemäß die Dauer der dem biologischen Objekt zugeführten Anregungsimpulsserie entsprechend einer Auswertung elektrophysikalischer Zustandsparameter des Zwischenelektrodenbereiches des biologischen Objektes verändert, welche gleichzeitig mit der Anregung des biologischen Objektes durchgeführt wird.

Es ist zweckmäßig, als elektrophysikalischen Parameter zur Auswertung des Zustandes des biologischen Objektes die Impedanz des Zwischenelektrodenbereiches des biologischen Objektes zu benutzen und diese Impedanz nach Zeitkennlinien eines elektrischen Signals zu bewerten, das nach der Beendigung jedes Anregungsimpulses entsteht.

Man kann auch die geänderte Dauer der Serie von Anregungsimpulsen mit der vorgegebenen Dauer vergleichen, und wenn die geänderte Seriendauer die vorgegebene unterschreitet, wird die Zuführung der nächsten Anregungsimpulsserie zum betreffenden Bereich des biologischen Objektes wiederaufgenommen, und wenn die geänderte Dauer der Anregungsimpulsserie gleich der vorgegebenen oder länger als diese ist, wird die Zuführung der Anregungsimpulsserie zum betreffenden Bereich des biologischen Objektes abgebrochen.

Es ist auch zweckdienlich, invertierte elektrische Impulse zusätzlich zu formieren, die für die Einwirkung auf den Bereich des biologischen Objektes bestimmt sind.

Diese Aufgabe wird auch dadurch gelöst, daß die Einrichtung zur Durchführung des Verfahrens zur Erzeugung von für die Anregung biologischer Objekte bestimmten elektrischen Impulsen, welche einen Impulsgenerator, einen Pulslängenmodulator, dessen einer Eingang am Ausgang des Impulsgenerators liegt, einen Leistungsverstärker für Anregungsimpulse, dessen Eingang mit dem Ausgang des Pulslängenmodulators verbunden ist und dessen Ausgänge an die Elektroden angeschlossen sind, die an einen Bereich des biologischen Objektes bei der Anregung gelegt werden, enthält, erfindungsgemäß einen ersten Former eines die Reaktion des biologischen Objektes auf den Anregungsimpuls bestimmenden Signals, dessen Eingang mit dem Ausgang des Anregungsimpuls-Leistungsverstärkers verbunden ist und an dessen Ausgang ein Signal für die Steuerung der Dauer der Anregungsimpulsserie gebildet wird, einen Generator für Impulse einer veränderbaren Dauer, die die Dauer der Anregungsimpulssserie festlegt und dessen Eingang mit dem Ausgang des ersten Formers eines die Reaktion des biologischen Objektes auf den Anregungsimpuls bestimmenden Signals verbunden ist, einen zweiten Former eines Signals, dessen Dauer der Dauer der Anregungsimpulsserie entspricht und das eine Vorder- und eine Hinterflanke aufweist, die sich mit einer konstanten Geschwindigkeit ändern, wobei der zweite Former eingangsseitig mit dem Ausgang des Generators für Impulse einer veränderbaren Dauer verbunden ist, und einen Höchstpegelbegrenzer für das Modulationssteuerungssignal enthält, dessen einer Eingang mit einer Signalpegelvorgabeschaltung, anderer Eingang mit dem Ausgang des zweiten Signalformers und dessen Ausgang mit dem zweiten Eingang des Pulslängenmodulators in Verbindung steht.

Die Einrichtung kann auch einen Zeitdiskriminator enthalten, an dessen Ausgang ein die Beendigung der Anregung anzeigendes Signal gebildet wird und dessen einer Eingang mit dem Ausgang des Generators für Impulse einer veränderbaren Dauer verbunden ist und dessen anderer Eingang am Ausgang einer Steuereinheit des Zeitdiskriminators liegt, die mit dem Ausgang des Anregungsimpuls-Leistungsverstärkers in Verbindung steht.

Der zweite Eingang der Steuereinheit des Zeitdiskriminators oder der dritte Eingang des Zeitdiskriminators kann mit dem Ausgang des Impulsgenerators verbunden sein.

Es ist zweckmäßig, daß die Einrichtung einen Zeitgeber, dessen Eingang mit dem Ausgang der Steuereinheit des Zeitdiskriminators verbunden ist, und einen steuerbaren Stromversorgungsteil enthält, der eingangsseitig mit dem Ausgang des Zeitgebers verbunden ist und die Stromversorgung abschaltet, und daß der zweite Eingang des Zeitgebers mit dem Ausgang des Impulsgenerators verbunden ist.

Es ist auch zweckdienlich, den Ausgang des Zeitdiskriminators mit dem dritten Eingang des Höchstpegelbegrenzers für das Modulationssteuerungssignal oder mit dem zweiten Eingang des steuerbaren Stromversorgungsteils zu verbinden.

Außerdem hat es sich als vorteilhaft erwiesen, daß die Einrichtung einen Umschalter der Elektroden aufweist, der zwischen den Elektroden und dem Anregungsimpuls-Leistungsverstärkers geschaltet ist.

Der erste Signalformer und der Generator für Impulse einer veränderbaren Dauer können als Multivibrator mit CMOS-Transistoren ausgebildet sein, in dessen erster Stufe in den Sources jedes Transistors Widerstände geschaltet sind, und am das Source des p-Kanal-Transistors ein Kondensator angeschlossen ist, dessen zweiter Anschluß den Eingang des ersten Signalformers bildet, wobei die erste Stufe des Multivibrators einen Rückkopplungskreis aus einer Diode und einem mit dieser in Reihe liegenden Widerstand aufweist, und der Ausgang der zweiten Multivibratorstufe dient als Ausgang des Generators für Impulse einer veränderbaren Dauer; der zweite Signalformer kann einen Integrator mit CMOS-Transistoren darstellen, bei denen zwischen den Speiseschienen und den Sources Widerstände geschaltet sind.

Auch ist es zweckmäßig, daß der Pulslängenmodulator einen aus CMOS-Transistoren aufgebauten Verstärker enthält, an dessen Eingang ein Differenzierkreis vorgesehen ist, dessen Zeitkonstante mit Hilfe eines einzelnen MOS-Transistors geändert wird, dessen Gate mit einem Eingang des Modulators verbunden ist, während der Eingang des Differenzierkreises als zweiter Eingang des Modulators dient.

Die Signalpegelvorgabeschaltung kann einen Integrator mit CMOS-Transistoren enthalten, bei denen zwischen Speiseschienen und den Sources Widerstände geschaltet sind, während der Eingang des Integrators über Umschalter mit den Speiseschienen in Verbindung stehen kann.

Es hat sich als bequem erwiesen, daß der Impulsgenerator einen Multivibrator mit CMOS-Transistoren darstellt, dessen erster Stufe einen aus CMOS-Transistoren aufgebauten Verstärker enthält, in dessen Rückkopplungskreis ein aus einem Widerstand und einem Kondensator bestehendes Trägheitsglied geschaltet ist, wobei zwischen den Speiseschienen und den Sources CMOS-Transistoren der ersten Stufe noch je ein zusätzlicher MOS-Transistor geschaltet ist und die zusammengeschalteten Gates der zusätzlichen MOS-Transistoren den Eingang der ersten Stufe bilden, während der Ausgang des Verstärkers als Ausgang der ersten Stufe des Multivibrators dient.

Es ist auch von Vorteil, daß die Steuereinheit des Zeitdiskriminators -in Reihe geschaltet-einen Widerstand und einen Negator, einen ersten Impulszähler, dessen Zähleingang mit dem Ausgang des Negators verbunden ist und dessen Steuereingang an dessen Ausgang angeschlossen ist, der Rücksetzeingang dieses Impulszählers als Eingang der Steuereinheit dient, und einen zweiten Impulszähler enthält, dessen Zähleingang mit dem Rücksetzeingang des ersten Impulszählers vereinigt ist, wobei der Steuereingang des zweiten Impulszählers mit seinem Ausgang und der Rücksetzeingang des zweiten Impulszählers mit dem Ausgang des ersten Impulszählers in Verbindung stehen.

Es ist wünschenswert, daß der Zeitdiskriminators ein ODER-Glied und einen Impulszähler enthält, dessen Rücksetzeingang mit dem Ausgang des ODER-Gliedes verbunden ist, wobei der Zähleingang dieses Zählers den Eingangs des Zeitdiskriminators bildet und der Steuereingang dieses Zählers mit seinem Ausgang verbunden ist.

Es ist auch erwünscht, daß der steuerbare Stromversorgungsteil eine Spannungsquelle und ein Flipflop enthält, dessen Setzeingang über einen Ausschalter mit dem Ausgang dar Spannungsquelle verbunden ist und dessen Rücksetzeingänge als Eingänge des steuerbaren Stromversorgungsteils dienen, wobei der Ausgang des Flipflops mit dem Steuereingang eines elektronischen Schalters verbunden ist, dessen Eingang an die Spannungsquelle angeschlossen ist.

Das erfindungsgemäße Verfahren und die Einrichtung zu seiner Durchführung ermöglichen eine individuell dosierte Anregung biologischer Gewebe, welche von elektrophysikalischen Parametern der Gewebe abhängt, unter minimaler äußerer energetischer Einwirkung und mit minimalem Energieaufwand.

Kurzbeschreibung der Erfindung

Im weiteren wird die Erfindung an Hand ihres Ausführungsbeispiels unter Bezugnahme auf die beigelegten Zeichnungen erläutert. Es zeigt:

Fig. 1a,b,c,d,e Zeitdiagramme elektrischer Impulse an den Elektroden;

Fig. 2 ein Strukturschaltbild einer erfindungsgemäßen Einrichtung zur Erzeugung von elektrischen Impulsen;

Fig. 3 ein Strukturschaltbild der Einrichtung mit einem Zeitdiskriminator gemäß der Erfindung;

Fig. 4 eine andere Ausführungsform der gleichen Einrichtung gemäß der Erfindung;

Fig. 5 eine weiters Ausführungsform der erfindungsgemäßen Einrichtung;

Fig. 6 ein Strukturschaltbild der Einrichtung mit einem Zeitgeber nach der Erfindung;

Fig. 7 eine Ausführungsform der erfindungsgemäßen Einrichtung mit Zeitgeber;

Fig. 8 ein Strukturbild der Einrichtung mit einer Variante der Begrenzung der Anregungsenergie gemäß der Erfindung;

Fig. 9 dieselbe Einrichtung mit Unterbrechung der Anregung;

Fig. 10 ein Strukturschaltbild der erfindungsgemäßen Einrichtung mit einem Umschalter der Elektroden;

Fig. 11 einen ersten Signalformer gemäß der Erfindung;

Fig. 12 einen zweiten Signalformer gemäß der Erfindung;

Fig. 13 einen Pulslängenmodulator gemäß der Erfindung;

Fig. 14 eine Signalpegelvorgabeschaltung gemäß der Erfindung;

Fig. 15 einen Impulsgenerator gemäß der Erfindung;

Fig. 16 eine Steuereinheit eines Zeitdiskriminators gemäß der Erfindung;

Fig. 17 einen Zeitdiskriminator gemäß der Erfindung;

Fig. 18 einen Stromversorgungsteil gemäß der Erfindung;

Fig. 19 einen Höchstpegelbegrenzer für das Signal gemäß der Erfindung;

Fig. 20 einen Leistungsverstärker gemäß der Erfindung;

Fig. 21 einen Zeitgeber gemäß der Erfindung:

Die beste Ausführungsform der Erfindung

Das Verfahren zur Erzeugung von für die Anregung biologischer Objekte bestimmten elektrischen Impulsen wird wie folgt durchgeführt.

Man erzeugt eine Folge von rechteckigen Impulse mit einer vorbestimmten Folgefrequenz, welche dann längenmoduliert werden, und formiert Serien reckteckiger Impulse mit einer vorgegebenen Folgefrequenz sowie mit einer vorgegebenen Pausenzeit zwischen den Serien. Dabei wird die Dauer der Impulse vorgegebener Frequenz in der Serie linear auf den Maximalwert verlängert, der entsprechend der individuellen Empfindlichkeit des biologischen Objektes eingestellt wird, bei der der Patient subjektive Empfindungen eines elektrischen Stechens komfortabler Art erleidet, und nachher linear auf Null vermindert.

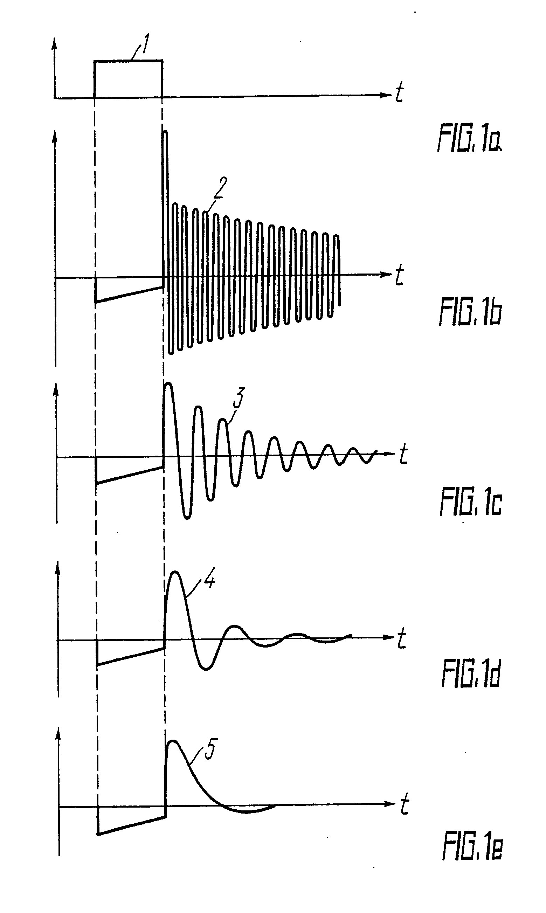

Die so erhaltenen Serien (Fig. 1a) von Anregungsimpulsen 1 werden verstärkt. Ein einzelner verstärkter Anregungsimpuls 2, der keinen Kontakt mit dem biologischen Objekt hat, ist in Fig. 1b dargestellt.

Die verstärkten Anregungsimpulse 1, welche Serien bilden, werden dem biologischen Objekt zugeführt. Dabei nehmen je nach dem Zustand elektrophysikalischer Parameter des Zwischenelektrodenbereiches des biologischen Objektes die Anregungsimpulse eine Form an, die jeweils in Fig. 1c,d,e gezeigt ist, worin Varianten der Anregungsimpulse mit schwachen, mittleren und ausgeprägten Abweichungen der elektrophysikalischen Parameter von der Norm dargestellt sind.

Als elektrophysikalischer Parameter, nach dem die Auswertung des Zustandes des biologischen Objektes durchgeführt, wird, kommt die Impedanz des Zwischenelektrodenbereiches des biologischen Objektes in Betracht.

Die Impedanz wird nach den Zeitkennlinien 3, 4, 5 (Fig. 1 c, d, e) eines elektrischen Signals bewertet, das nach der Beendigung jedes Anregungsimpulses 1 auftritt. Darauf verändert man entsprechend der so erhaltenen Auswertung der Impedanz die Dauer der Anregungsimpulsserie und vergleicht einen erhaltenen Wert der Seriendauer mit der vorgegebenen Seriendauer.

Unterschreitet dabei die erhaltene Seriendauer die vorgegebene, so wird die Zuführung von Anregungsimpulsserien wiederaufgenommen, und wenn der erhaltene Wert der Seriendauer länger als die vorgegebene oder dieser gleich ist, wird die weitere Zuführung von Anregungsimpulsserien zu dem betreffenden Bereich (Abschnitt) des biologischen Objektes abgebrochen.

Um den therapeutischen Effekt der elektrischen Anregung zu erhöhen, werden die obenaufgezählten Verfahrensschritte wiederholt, wobei man die Einwrikungsimpulse invertiert.

Ausgehend von der Dynamik der Änderungen von Störungen unter Einwirkung einer Anregungsimpulsserie bestimmt man einen Bereich für die Durchführung der nachfolgenden Anregung, was die Möglichkeit bietet, die äußere energetische Einwirkung herabzusetzen und die Zeit für die Prozeßführung zu reduzieren, was dementsprechend zu einer allmählichen Verkleinerung der Fläche, auf die eingewirkt wird, führt.

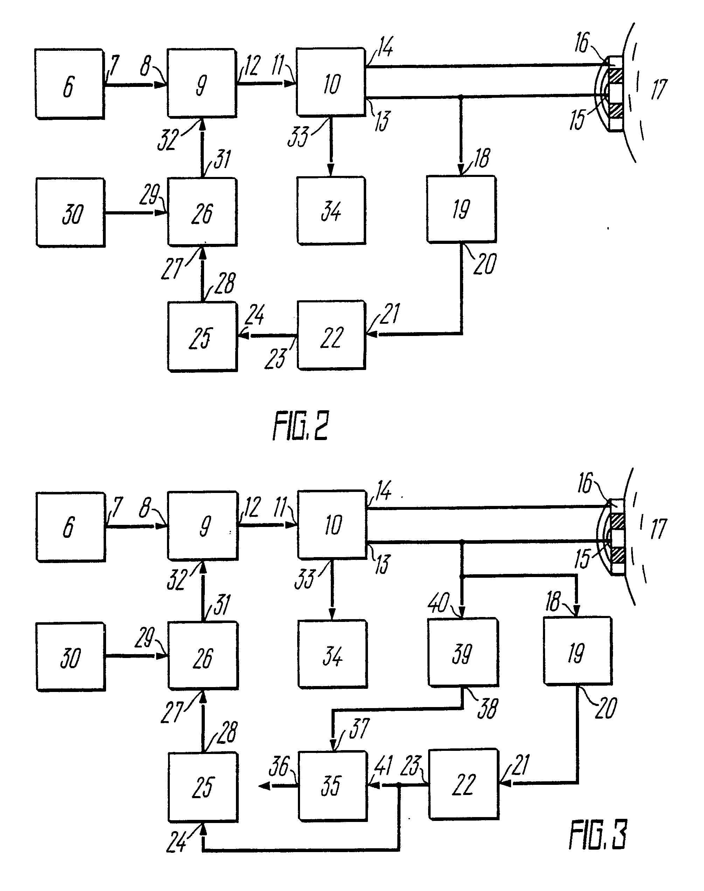

Die Einrichtung zur Erzeugung von für die Anregung bestimmten elektrischen Impulsen enthält einen Impulsgenerator 6 (Fig. 2), dessen Ausgang 7 am Eingang 8 eines Pulslängenmodulators 9 liegt, einen Anregungsimpuls-Leistungsverstärker 10, dessen Eingang 11 am Ausgang 12 des Modulators 9 liegt und dessen Ausgänge 13, 14 mit Elektroden 15, 16 verbunden sind, welche entsprechenderweise an einen Bereich 17 eines biologischen Objektes bei der Anregung gelegt werden.

An den Ausgang 13 des Verstärkers 10 ist der Eingang 18 eines Formers 19 eines die Reaktion des biologischen Objektes auf einen Anregungsimpuls bestimmenden Signals angeschlossen. Am Ausgang 20 des Formers 19 wird ein Signal für die Steuerung der Dauer einer Anregungsimpulsserie formiert. Der Ausgang 20 des Formers 19 steht mit dem Eingang 21 eines Generators 22 für Impulse einer veränderbaren Dauer, die die Dauer der Anregungsimpulse festlegt, in Verbindung, dessen Ausgang 23 mit dem Eingang 24 eines Formers 25 eines Signals verbunden ist, dessen Dauer der Dauer der Anregungsimpulsserie entspricht und das eine Vorder- und eine Hinterflanke aufweist, die sich mit einer konstanten Geschwindigkeit ändern.

Die Einrichtung hat auch einen Höchstpegelbegrenzer 26 für das Modulationssteuerungssignal, dessen Eingang 27 mit dem Ausgang 28 des Signalformers 25 und dessen Eingang 29 mit einer Signalpegelvorgabeschaltung 30 verbunden ist. Der Ausgang 31 des Begrenzers 26 ist mit dem zweiten Eingang 32 des Pulslängenmodulators 9 verbunden. An den Ausgang 33 des Leistungsverstärkers 10 ist ein Anzeiger 34 geschaltet.

Die Einrichtung ist auch mit einem Zeitdiskriminator 35 versehen (Fig. 3), an dessen Ausgang 36 ein Signal für das Abbrechen (die Begrenzung) der Anregung gebildet wird und dessen Eingang 37 mit dem Ausgang 38 einer Steuereinheit 39 des Zeitdiskriminators 35 verbunden ist. Der Eingang 40 der Steuereinheit 39 des Zeitdiskriminators 35 ist an den Ausgang 13 des Leistungsverstärkers 10 angeschlossen, wobei der Eingang 41 des Zeitdiskriminators 35 mit dem Ausgang 23 des Generators 22 für Impulse einer veränderbaren Dauer verbunden ist.

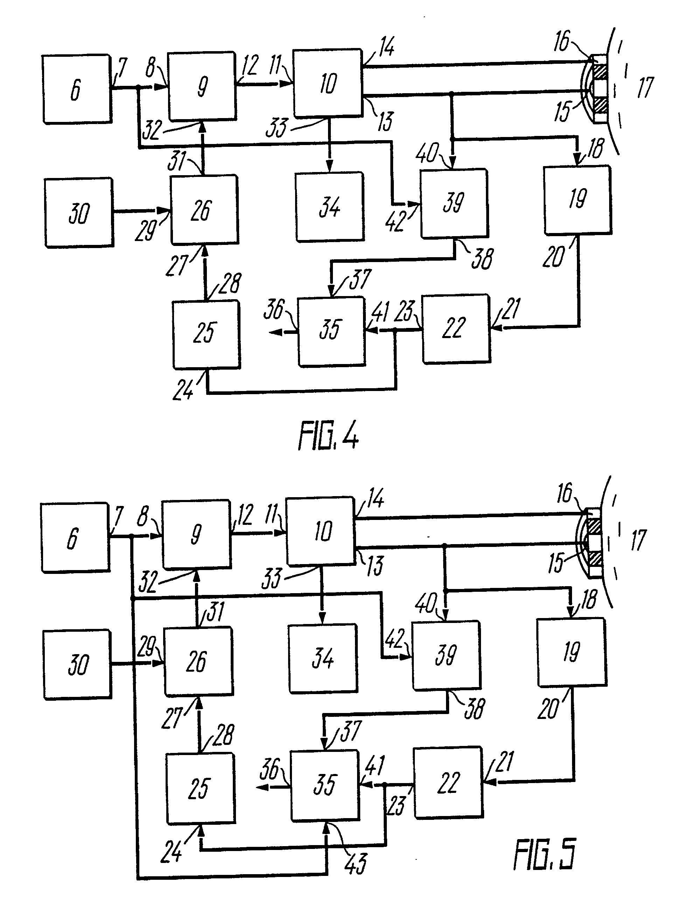

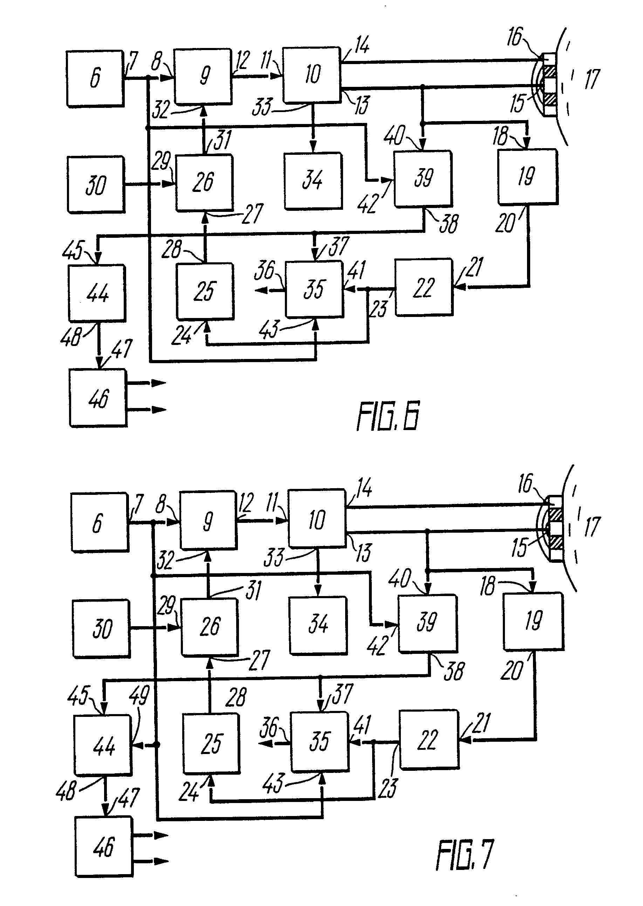

Der Eingang 42 (Fig. 4) der Steuereinheit 39 des Zeitdiskriminators 35 und der Eingang 43 (Fig. 5) des letzteren sind an den Ausgang 7 (Fig. 4, 5) des Impulsgenerators 6 angeschlossen.

Die Einrichtung weist auch einen Zeitgeber 44 (Fig.6), dessen Eingang 45 an den Ausgang 38 der Steuereinheit 39 des Zeitdiskriminators 35 geführt ist, und einen steuerbaren Stromversorgungsteil 46 auf, dessen Eingang 47 mit dem Ausgang 48 des Zeitgebers 44 verbunden ist, wobei der Eingang 49 (Fig. 7) des Zeitgebers 44 mit dem Ausgang 7 des Impulsgenerators 6 in Verbindung steht.

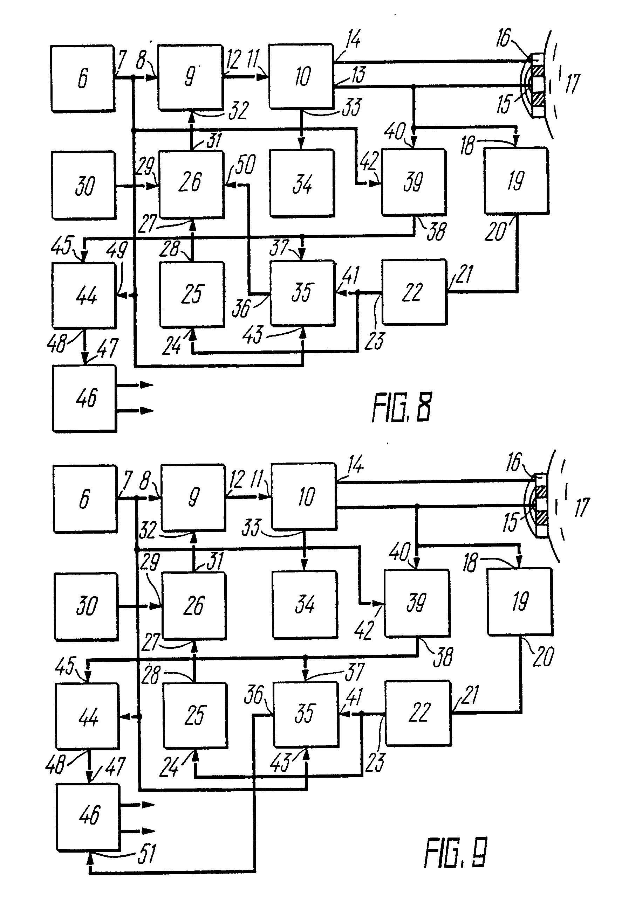

Der Ausgang 36 (Fig. 8) des Zeitdiskriminators 35 ist an den Eingang 50 des Höchstpegelbegrenzers 26 für das Modulationssteuerungssignal angeschlossen.

Bei einer anderen Ausführungsform der Einrichtung ist der Ausgang 36 des Zeitdiskriminators 35 mit dem Eingang 51 des Stromversorgungsteils 46 verbunden.

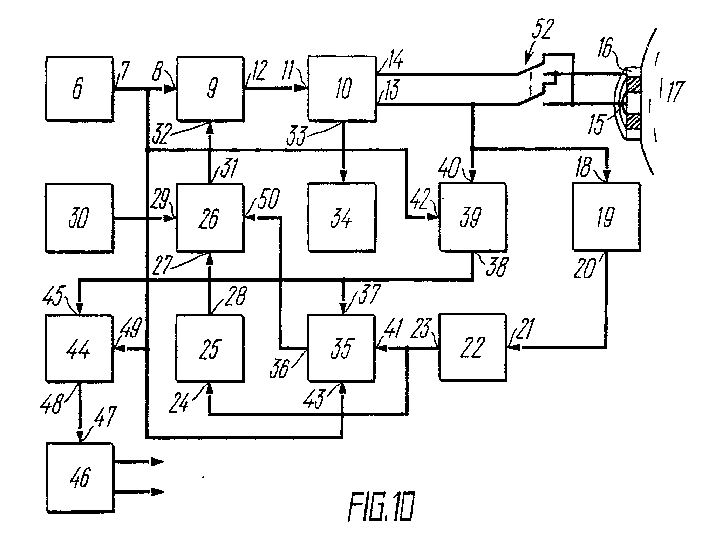

In den beiden Ausführungsformen der Einrichtung enthält diese einen Umschalter 52 (Fig. 10) der Elektroden, der zwischen den Ausgängen 13, 14 des Leistungsverstärkers 10 und den Elektroden 15, 16 geschaltet ist und zwei Schaltstellungen hat, welche die Möglichkeit bieten, an jeden Ausgang 13 (14) des Verstärkers 10 eine jede der Elektroden 15 (16) anzuschließen.

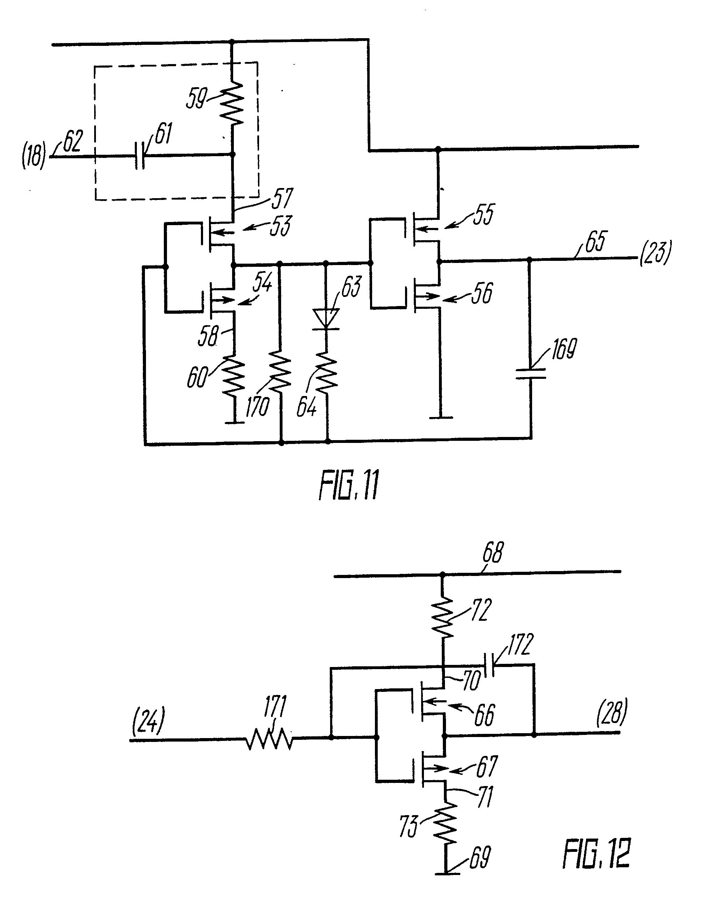

Bei der Einrichtung sind der erste Former 19 und der Generator 22 für Impulse einer veränderbaren Dauer auf der Basis eines Multivibrators mit CMOS-Transistoren 53 (Fig.11), 54 und 55, 56 ausgeführt, bei welchem Multivibrator in den Source-Elektroden 57 und 58 der Transistoren 53 bzw. 54 der ersten Stufe jeweils Widerstände 59 und 60 geschaltet sind, und an das Source 57 des Transistors 53 ist ein Kondensator 61 angeschlossen, dessen zweiter Anschluß 62 den Eingang 18 (Fig. 2) des ersten Formers 19 bildet. Die erste Stufe weist einen Rückkopplungskreis aus einer Diode 63 (Fig. 11) und einem mit dieser in Reihe liegenden Widerstand 64 auf, während der Ausgang 65 der zweiten Stufe des Multivibrators als Ausgang 23 (Fig. 2) des Generators 22 für Impulse einer veränderbaren Dauer dient.

Der zweite Signalformer 25 stellt einen Integrator mit CMOS-Transistoren 66 (Fig. 12) und 67 dar, zwischen deren Speiseschienen 68 und 69 und den Sources 70 und 71 jeweils Widerstände 72 und 73 geschaltet sind.

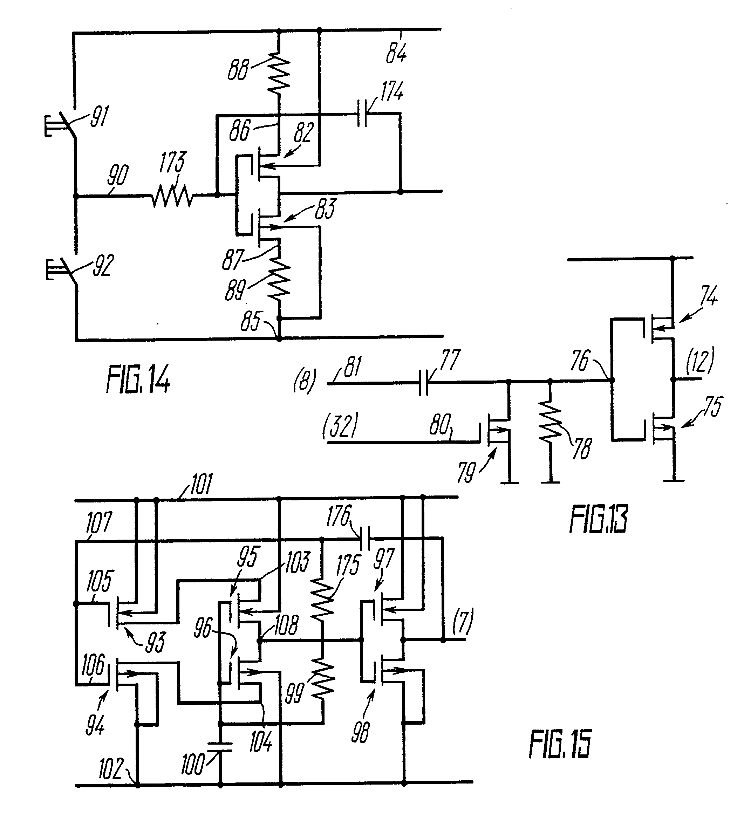

Der Pulslängenmodulator 9 (Fig. 2) enthält einen aus CMOS-Transistoren 74 (Fig. 13) und 75 aufgebauten Verstärker, an dessen Eingang 76 ein Differenzierkreis aus einem Kondensator 77 und einem Widerstand 78 vorgesehen ist, dessen Zeitkonstante sich mit Hilfe eines einzelnen MOS-Transistors 79 ändert, dessen Gate den Eingang 32 (Fig. 2) des Pulslängenmodulators 9 bildet, wobei der Eingang 81 (Fig.13) des Differenzierkreises als Eingang 8 (Fig. 2) des Pulslängenmodulators dient.

Die Signalvorgabeschaltung 30 enthält einen Integrator mit CMOS-Transistoren 82 (Fig. 14) und 83, zwischen deren Speiseschienen 84 und 85 und deren Sources 86 und 87 jeweils Widerstände 88 und 89 geschaltet sind, während der Eingang 90 des Integrators über Umschalter 91 und 92 mit den Speiseschienen 84 bzw. 85 verbunden ist.

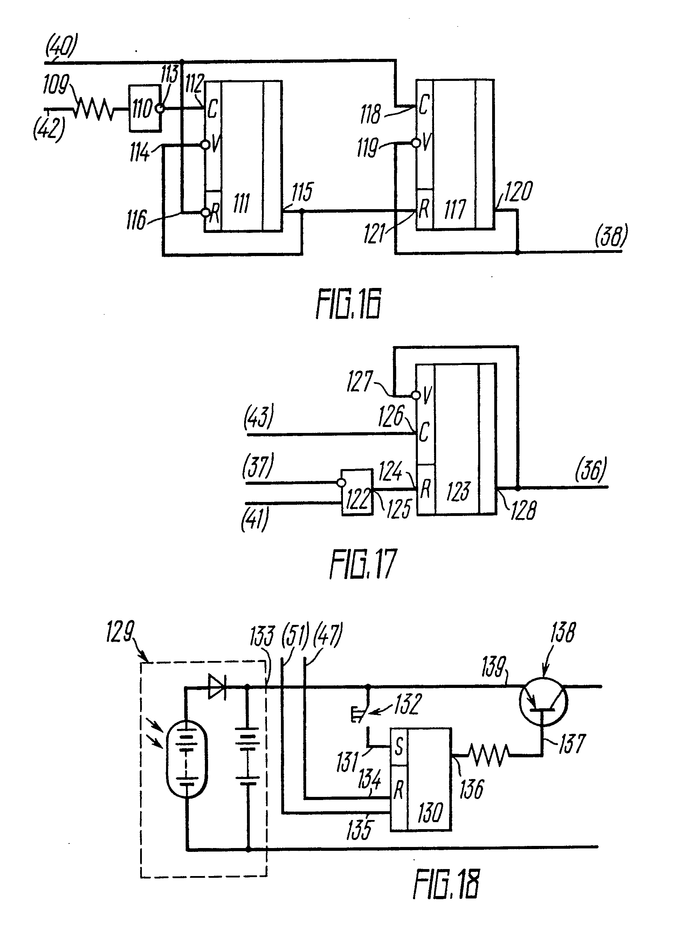

Der Impulsgenerator 6 (Fig. 2) stellt einen Multivibrator mit CMOS-Transistoren 93 (Fig. 15), 94,95,96,97,98 dar, in dessen erster Stufe ein Verstarker mit CMOS-Transistoren 95,96 enthalten ist und im Rückkopplungskreis ein Trägheitsglied aus einem Widerstand 99 und einem Kondensator 100 geschaltet ist, wobei zwischen den Speiseschienen 101, 102 und den Sources 103,104 der CMOS-Transistoren 95 bzw. 96 zusätzliche MOS-Transistoren 93,94 geschaltet sind.

Die zusammengeschalteten Gates 105, 106 der MOS-Transistoren 93, 94 bilden dabei den Eingang 107 der ersten Multivibratorstufe, während der Ausgang 108 des aus CMOS-aufgebauten Verstärkers 95,96 als Ausgang der ersten Multivibratorstufe dient.

Die Steuereinheit 39 (Fig. 3) des Zeitdiskriminators 35 enthält -in Reihe geschaltet- einen Widerstand 109 (Fig. 16) und einen Negator 110, einen ersten Impulszähler 111,dessen Zähleingang 112 mit dem Ausgang 113 des Negators 110 verbunden ist und dessen Steuereingang 114 mit dessen Ausgang 115 in Verbindung steht.

Der Rücksetzeingang 116 des Impulszählers 111 ist der Eingang 40 (Fig. 3) der Steuereinheit 39 des Zeitdiskriminators 35. Die Steuereinheit 39 des Zeitdiskriminators 35 enthält auch einen zweiten Impulszähler 117 (Fig. 16), dessen Zähleingang 118 mit dem Rücksetzeingang 116 des Impulszählers 111 vereinigt ist. Der Steuereingang 119 des Impulszahlers 117 steht mit dessen Ausgang 120 in Verbindung, während der Rücksetzeingang 121 des Zählers 117 am Ausgang 115 des Impulszählers 111 liegt.

Der Zeitdiskriminator 35 (Fig. 3) enthält ein ODER-Glied 122 (Fig.17) und einen Impulszähler 123, dessen Rücksetzeingang mit dem Ausgang 125 des ODER-Gliedes 122 verbunden ist. Der Zähleingang 126 des Impulszählers 123 bildet den Eingang 43 (Fig. 3) des Zeitdiskriminators 35, während oder Steuereingang 127 (Fig. 17) des Impulszählers 123 mit dessen Ausgang 128 verbunden ist.

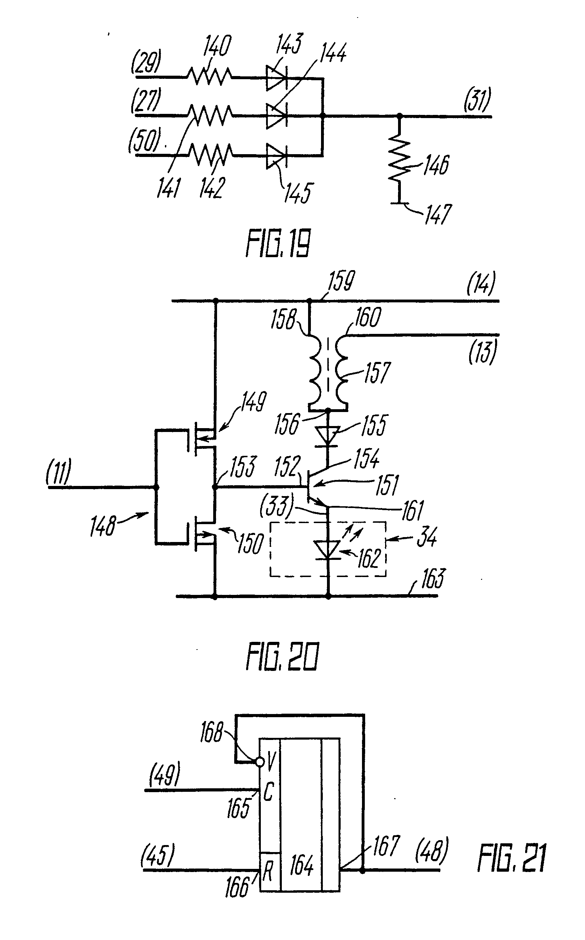

Der Stromversorgungsteil 46 (Fig. 6) enthält eine Spannungsquelle 129 (Fig. 18) und ein Flipflop 130, dessen Setzeingang 133 über einen Ausschalter 132 mit dem Ausgang 133 der Spannungsquelle 129 verbunden ist. Die Eingänge 134, 135 des Flipflops 130 stellen Eingänge 47 (Fig. 6), 51 des Stromversorgungsteils 46 dar, während der Ausgang 136 (Fig. 18) des Flipflops 130 mit dem Steuereingang 137 eines elektronischen Schalters 138 verbunden ist, dessen Eingang 139 an die Spannungsquelle 129 gelegt ist.

Die Spannungsquelle 129 kann als Satz von galvanischen Elementen und Sonnenenergiequellen ausgeführt werden, während der elektronische Schalter 138 auf einem Transistor aufgebaut ist.

Der Höchstpegelbegrenzer 26 (Fig. 2) für das Modulationssteuerungssignal enthält drei Stromkreise, die jeweils eine Reihenschaltung eines Widerstandes 140 (Fig. 19)), 141, 142 und einer Diode 143, 144 bzw. 145 darstellen. Die anderen Anschlußenden der Dioden 143,144,145 sind vereinigt und mit dem Ausgang 31 (Fig. 2) des Höchstpegelbegrenzers 26 für das Modulationssteuerungssignal verbunden und über einen Widerstand 146 (Fig. 19) an eine gemeinsame Sammelschiene 147 gelegt.

Der Leistungsverstärker 10 (Fig. 2) und der Anzeiger 34 enthält einen Verstärker 148 (Fig. 20) mit CMOS-Transistoren 149, 150 und einen Transistor 151, dessen Basis 152 an den Ausgang 153 des Verstärkers 148 angeschlossen ist. Der Kollektor 154 des Transistors 151 ist über eine Diode 155 an eine Abzweigung 156 einer Induktivitätsspule 157 angeschlossen. Das erste Anschlußende 158 der Spule 157 ist an eine Speiseschiene 159 angeschlossen, während das zweite Anschlußende 160 den ersten Ausgang 13 (Fig. 2) des Leistungsverstärkers 10 bildet. Der zweite Ausgang 14 des Leistungsverstärkers 10 ist mit der Speiseschiene 159 (Fig. 20) verbunden. Der Emitter 161 des Transistors 151 steht mit dem dritten Ausgang 33 (Fig. 2) des Leistungsverstärkers 10 in Verbindung und ist an den Anzeiger 34 geschaltet, der eine Diode 162 (Fig. 20) enthält, die an die gemeinsame Sammelschiene 163 angeschlossen ist.

Der Zeitgeber 44 (Fig. 7) enthält einen Impulszahler 164 (Fig. 21), dessen Zähleingang 165 mit dem Eingang 49 (Fig.7) des Zeitgebers 44 und dessen Rücksetzeingang 166 (Fig. 21) mit dem Eingang 44 (Fig. 7) des Zeitgebers 44 verbunden ist. Der Ausgang 167 (Fig.21) ist an den Steuereingang 168 des Zählers 164 und an den Ausgang 48 (Fig. 7) des Zeitgebers 44 geschaltet.

Bei der Behandlung der Realisierungsbeispiele der einzelnen Einheiten der Einrichtung wurden einige Bauelemente dieser Einheiten nicht erwähnt. So enthält beispielsweise der Signalformer 19 (Fig. 2) einen zeitbestimmenden Kondensator 169 (Fig. 11) und einen Widerstand 170; der Former 25 (Fig. 2) ist mit einem Widerstand 171 (Fig. 12) und einem Kondensator 172 versehen; die Schaltung 30 (Fig. 2) zur Signalpegelvorgabe (Fig. 2) weist einen zeitbestimmenden Widerstand 173 (Fig. 14) und einen Kondensator 174 auf, während der Impulsgenerator 6 (Fig. 2) einen zeitbestimmenden Widerstand 175 (Fig. 15) und einen Kondensator 176 aufweist.

Die Einrichtung nach Fig. 10 funktioniert wie folgt.

Die Einrichtung wird durch kurzzeitiges Schließen des Ausschalters 132 im Stromversorgungsteil 46 eingeschaltet. Dabei wird mit dem am Ausgang 136 des Flipflops 130 erscheinenden Signal der elektronische Schalter 138 geschlossen und die Versorgungsspannung allen Baugruppen der Einrichtung zugeführt.

Der Impulsgenerator 6 wirkt analog dem bekannten, für den gleichen Zweck bestimmten Generator, hat aber einen geringeren Energieverbrauch. Am Ausgang 7 des Generators 6 wird eine Folge von rechteckigen Impulsen mit einer vorgegebenen Folgefrequenz gebildet, die dann zum Eingang 8 des Modulators 9 gelangt.

Mit dem Modulator 9 wird die Modulation der eintreffenden Impulse in der Länge durchgeführt, wobei ihre Dauer proportional zum Pegel des am Eingang 32 des Modulators 9 anliegenden Signals bis auf Null verkürzt wird. Die Wirkungsweise des Modulators 9 ist ähnlich der des bekannten, für den gleichen Zweck bestimmten.

Die längenmodulierten Anregungsimpulse gelangen vom Ausgang 12 des Modulators 9 an den Eingang 11 des Leistungsverstärkers 10.

Die Induktivitätsspule 157 im Verstarker 10 stellt einen Aufwartstransformator dar und sichert damit die Erzeugung von Hochspannungsimpulsen am Ausgang 13 des Verstarkers 10, welche danach zur Elektrode 15 gelangen. Die Elektrode 16 ist indifferent und steht über den Ausgang 14 des Verstärkers 10 mit der Speiseschiene 159 in Verbindung. Die Dauer der Impulse an der Elektrode 15 wird nach der Leuchtintensität des Anzeigers 34 bewertet.

Im Ausgangszustand formiert der Generator 22 am Ausgang 23 Rechteckimpulse, deren Dauer die Seriendauer festlegt.

Der Generator 22 hat die Möglichkeit, die Dauer der zu erzeugenden Impulse entsprechend dem an seinem Eingang 21 anliegenden Signal ohne Änderung der Pausenzeit zwischen den Impulsen zu verändern.

Die Impulse vom Ausgang 23 des Generators 22 treffen am Former 25 ein, der für die Bildung der an dessen Ausgang 28 auftreffenden Impulse mit linearer ansteigender Vorder- und linear abfallender Hinterflanke sorgt.

Die gebildeten Impulse treffen am Eingang 27 des Begrenzers 26 ein, der die Begrenzung der Amplitude der so gebildeten Impulse auf einen Wert gewährleistet, der durch die Schaltung 30 am Eingang 29 des Begrenzers 26 vorgegeben wird.

Dabei wird die Vergrößerung oder Verminderung des durch die Schaltung 30 gebildeten Spannungspegels durch das Schließen des Umschalters 92 oder 91 gewährleistet.

Auf solche Weise erscheinen am Ausgang 31 des Begrenzers 26 negative Impulse mit linear veränderlicher Vorder- und Hinterflanken, mit einer Dauer, die der Seriendauer entspricht, und mit einer vorgegebenen Pausenzeit zwischen den Impulsen und einem minimalen Pegelwert, der durch den Spannungspegel am Eingang 29 des Begrenzers 26 bestimmt wird.

Die so begrenzten Impulse treffen am Eingang 32 des Modulators 9 ein, an dessen Ausgang 12 Serien rechteckiger Anregungsimpulse mit linearer Verlängerung der Dauer der Impulse in einer Serie auf den (mit Hilfe der Schaltung 30) vorgegebenen Wert und mit einer vorgegebenen Pausenzeit zwischen den Anregungsimpulsserien gebildet werden.

Stehen die Elektroden 15 und 16 in Keinem Kontakt mit dem Bereich 17 des biologischen Objektes, zeichnen sich die Impulse 2 (Fig. 1b) am Ausgang 13 des Verstärkers 10 durch eine hohe Schwingungsfrequenz und eine relativ kleine Dämpfung aus. Dabei reicht das Signal am Ausgang 20 des Formers 19 für die Änderung der Betriebsart des Generators 22 nicht aus.

Die Steuereinheit 39 des Zeitdiskriminators 35 gewährleistet eine Aussonderung der Schwingungsfrequenz am Eingang 40. Die Schaltung 39 wirkt analog der für diesen Zweck bestimmten bekannten Schaltung, ist aber zur Verbesserung der Genauigkeit der Frequenzunterscheidbarkeit aus digitalen Bauelementen aufgebaut und wird mit einer Impulsfolge vom Ausgang 7 des Generators 6 synchronisiert.

Somit wird beim Anliegen einer Hochfrequenz (z.B. von Impulsen 2 in Fig. 1b) am Eingang 40 der Schaltung 39 ein Signal an deren Ausgang 38 gebildet, durch das die Funktion des Zeitdiskriminators 35 verboten und die Funktion des Zeitgebers 44 freigegeben wird.

Der Zeitgeber 44 ist zur Erhöhung der Genauigkeit bei der Bildung eines Zeitintervalls aus digitalen Bauelementen aufgebaut und wird mit einer Impulsfolge vom Ausgang 7 des Generators 6 synchronisiert.

Liegt innerhalb des im Zeitgeber 44 eingestellten Zeitintervalls eine Hochfrequenz an, was demjenigen Zustand der Einrichtung entspricht, bei dem sie mit dem Bereich 17 des biologischen Objektes in keinem Kontakt steht, so wird am Ausgang 48 des Zeitgebers 44 ein Signal formiert, das zum Eingang 47 des Stromversorgungsteils 46 gelangt und die ganze Einrichtung ausschaltet.

Sobald die Elektroden 15, 16 mit dem Bereich 17 des biologischen Objektes in Berührung gebracht sind, erscheinen an diesen Elektroden je nach dem Zustand des biologischen Objektes 17 Signale, die den Kennlinien 3,4 oder 5 (Fig. 1c, d,e) entsprechen. Hierbei wird am Ausgang 20 des Formers 19 ein Signal gebildet, das für die Änderung der Dauer des vom Generator 22 erzeugten Impulses ausreicht. Da sich der am Eingang 40 der Schaltung 39 ablaufende Vorgang durch eine relativ niedrige Frequenz auszeichnet, wird am Ausgang 38 der Schaltung 39 ein Signal gebildet, durch das der Zeitgeber 44 zurückgesetzt und seine Funktion verboten sowie gleichzeitig die Arbeit des Zeitdiskriminators 35 freigegeben wird.

Durch den Zeitdiskriminator 35 wird die Dauer der vom Generator 22 zu erzeugenden Impulsen mit der vorgegebenen Dauer verglichen, wobei der Zeitdiskriminator 35 analog dem für den gleichen Zweck geeigneten bekannten funktioniert, aber aus digitalen Bauelementen zwecks Erhöhung der Genauigkeit der Erzeugung von Zeitintervallen aufgebaut ist und mit einer Impulsfolge vom Ausgang 7 des Generators 6 synchronisiert wird.

Da im Prozeß der Einwirkung auf das biologische Objekt sich dessen Zustand und folglich die Charakteristik des Prozesses (Fig. 1) am Ausgang 13 des Verstärkers 10 ändern, ergibt sich von einem gewissen Zeitpunkt an ein solches Signal am Ausgang 20 des Formers 19, bei welchem die Dauer der vom Generator zu erzeugenden Impulse gleich oder größer als die vorgegebene wird. Hierbei wird am Ausgang 36 des Zeitdiskriminators 35 ein Signal formiert, das über den Begrenzer 26 auf den Steuereingang 32 des Modulators einen Einfluß ausübt, so daß auf einen gewissen Mindestwert die Dauer der Anregungsimpulse am Ausgang 12 des Modulators verkürzt wird und damit die Möglichkeit einer Überdosierung der Behandlungseinwirkung kleiner wird.

Der stationäre Betrieb der Einrichtung wird beibehalten, bis die Elektroden 15, 16 mit dem biologischen Objekt in Berührung stehen. Der Ausgangszustand der Einrichtung wird automatisch, sobald die Elektroden 15, 15 mit dem biologischen Objekt außer Berührung gebracht werden, wiederhergestellt, wie es oben beschrieben wurde.

Um den therapeutischen Effekt zu erhöhen, wird zwischen den Ausgängen 13, 14 und den Elektroden 15, 16 ein Umschalter 52 geschaltet, mit dessen Hilfe die Umschaltung der aktiven und indifferenten(unwirksamen) Elektrode 15 bzw. 16 auf die Ausgänge 13 und 14 erfolgt.

Der Unterschied der Arbeitsweise der Einrichtung gemäß Fig. 9, 11 bis 21 von der früher beschriebenen besteht darin, daß das Signal vom Ausgang 36 des Zeitdiskriminators 35 am Eingang des Stromversorgungsteils 46 eintrifft und die Stromversorgung der Einrichtung abschaltet, wodurch eine Überdosierung der Einwirkung auf einen konkreten Bereich 17 des biologischen Objektes vermieden wird.

Somit wird der Ausgangszustand der Einrichtung beim Aufnören des Kontaktes zwischen den Elektroden 15, 16 und dem biologischen Objekt nicht wiederhergestellt und bedarf einer Wiedereinschaltung der Einrichtung.

Die Wirkungsweise der in Fig. 2 bis 8 dargestellten Einrichtungen ist analog der obenbeschriebenen, weil sie verschiedene Ausführungsformen darstellen.

Das erfindungsgemäße Verfahren und die zugehörige Einrichtung wurden klinischen Prüfungen unterworfen, deren Ergebnisse unten dargelegt werden.

Die erfindungsgemäße Methodik wurde an einer großen Gruppe von Kranken mit Erkrankungen des peripheren Nervensystems, mit Wurzel- und neuritischen Veränderungen, statischen und dynamischen Störungen, mit Erkrankungen des Stütz- und Bewegungsapparates verschiedener Ätiologie (entzündliche, metabolische, genetische und andere Erkrankungen), Erkrankungen des Zentralnervensystems mit liquordynamischen Störungen sowie mit Störungen und Störungsfolgen im zerebralen Kreislauf, mit entzündlichen Erkrankungen der oberen Luftwege, mit Krankheiten im Magen-Darm-Kanal, mit Erkrankungen des vegetativen Nervensystems und anderen pathologischen Zustanden bei gestörten Adaptationsprozessen angewendet.

Unter den Erkrankungen des peripheren Nervensystems kamen Osteochondrosen der Hals- und Lendenwirbelsaule am meisten vor. Die Exazerbationsdauern betrugen vor Beginn der Behandlung von 3... 5 Tagen bis 2,5 ... 3 Monate, die Erkrankungsdauer waren ebenfalls unterschiedlich. Es wurde folgendes festgestellt. Die Einwirkungszeit sei streng individuell. Sie war kürzer bei einer geringeren Exazerbationsdauer und guten kompensatorischen Möglichkeiten des Organismus des Kranken. Die Einwirkungszeit wurde verlängert bei eine längere Zeit dauernden Exazerbationen (Rezidiven) vor der Behandlung oder bei einer längeren Erkrankungsdauer.

Es wurde eine kennzeichnende Wirkung an einer Kontrollgruppe aus 10 Menschen, die einer komplexen Therapie, einschließlich einer Nadelreflextherapie unterworfen wurden, erhalten. Positive Veränderungen zeigten sich nicht nur in der Verminderung organischer und funktioneller Herdveränderungen sondern auch betrafen den psychoemotionellen Zustand. Es wurde die Einwirkung örtlich, segmentär ausgeübt. Die Anzahl von Behandlungsprozeduren betrug 3 bis 15 jeden zweiten Tag oder zweimal in der Woche. Als Kontingent der Kranken kamen ambulante Patienten und Kranken der neuralgischen Krankenhausabteilung in Betracht.

Die Anwendung des erfindungsgemäßen Verfahrens verkürzt die Behandlungsdauer sowohl unter ambulanten als auch unter den Krankenstationsverhältnissen sowie bei selbständiger (häuslicher) Behandlung oder im Komplex mit anderen Behandlungsverfahren. Es war eine gute Verträglichkeit der Einwirkung zu verzeichnen, wobei keine Zustandsverschlimmerung der Kranken nicht beobachtet wurde. Positive Veränderungen wurden bei der Behandlung von anderen Pathologiearten festgestellt.

In sechs Fällen wurde der Effekt "Placebo" geprüft, die elektrische Anregung wurde mit Hilfe von anderen bekannten ähnlichen Einrichtungen durchgeführt. Festgestellt wurde in sämtlichen sechs Fällen die Erkennung einer Placebo-Wirkung nach Unterscheidungsmerkmalen und Effekten, die nach der Anregung auftreten.

Um die Kriterien für die individuell dosierte Anregung zu beweisen, wurden folgende klinische Untersuchungen vorgenommen.

Es wurden 20 Patienten mit Erkrankungen des peripheren Nervensystems, mit Wurzelveränderungen ausgewählt, bei denen gleiche statische und dynamische Störungen beobachtet wurden, die Anfangszeit des nächstfolgenden Rezidivs bis 3 Tage betrug, die Erkrankungsdauer bis 5 Jahre ausmachte, wobei dis Nerventätigkeit der Patienten von sanguinischem und cholerischem Typ war. Unter den Kranken waren 12 Männer und 8 Frauen. Sie bildeten drei Gruppen.

Die erste Gruppe setzte sich aus 8 Menschen- 5 Männern und 3 Frauen- zusammen. Es wurde eine Einwirkung auf die Bereiche der Hautdecke über dem Gebiet erkrankter Wirbel und im paraverteralen Bereich ausgeübt entsprechend der Auswertung der Impendanz des Bereiches des Gewebes, bis die Resultate stabil wurden, wobei nur bei einem Vorzeichen der Elektrodenpolarisation gearbeitet wurde (es wurde die Methodik einer individuell dosierten Anregung verwendet).

In der zweiten Gruppe aus 6 Menschen - 2 Männern und 4 Frauen- wurde die individuell dosierte Anregung analog, aber bei verschiedenen Polungen der Elektroden durchgeführt.

In der dritten Gruppe aus 6 Menschen- 5 Männern und einer Frau -wurde nach der individuell dosierten Anregung eine zwangsmäßige Einwirkung (doppelte Zeit der dosierten Anregung bei gleichem Vorzeichen der Polung) ausgeübt.

Die Kontrolle statischer und dynamischer Veränderungen und von Wurzelstörungen wurde vor, nach der Anregung und nach jeder Einwirkung vorgenommen.

In sämtlichen Gruppen wurde ein positiver therapeutischer Effekt erreicht, der in der Verbesserung von statischen und dynamischen Indizes der Wirbelsäule, in der Verminderung von Wurzelprolapsen zum Ausdruck gebracht wurde und der nach der Behandlungsprozedur erhältenblieb und zum Zeitpunkt der nächstfolgenden Einwirkung zunahm. Der therapeutische Effekt vereinigte sich mit einer Änderung des emotionalen Hintergrundes und des gesamten Zustandes der Kranken. Ein besonders ausgepragter klinischer Effekt wurde bei den Kranken der zweiten Gruppe festgestellt. Diese Kranken benötigten weniger Behandlungsprozeduren ( 3 bis 4 ), um den arbeitsfahigen Zustand zu erreichen, gegenüber den Kranken der ersten und der dritten Gruppe, welche dafür 4 bis 5 bzw. 6 bis 10 Prozeduren brauchten.

In der dritten Gruppe wurde eine Verminderung der nach der dosierten Anregung erreichten klinischen Veränderungen (bis 5 ...7%) nach der erzwungenen Anregung beobachtet.

Es wurden alle Kranken nach 6 bis 7 Monaten dynamisch untersucht, wobei keine Rezedive in allen Gruppen beobachtet wurden. Bei den Kranken der dritten Gruppe wurden periodische unangenehme Paresen festgestellt, die von unbedeutenden Beschränkungen der Beweglichkeit der Wirbelsäule begleitet wurden.

Das erfindungsgemäße Verfahren kann zur elektrischen Heilanregung mit individueller Dosierung verwendet werden, wobei die äußere energetische Einwirkung verringert, die Zeit der Einwirkung individuell eingestellt und die Zeit der nachfolgenden Prozeduren durch Wahl der Einwirkungsstelle verkürzt wird. Die Einrichtung realisiert das Verfahren im automatischen Betrieb und hat einen geringen Energieverbrauch. Die durch die Einrichtung ausgeübte Einwirkung beeinflußt die Kompensations- und Adaptationsvorgänge im Organismus.

Es werden die Behandlungsdauern beträchtlich und die Verbleibzeit im krankenhaus um 5 bis 10 Tags und mehr verkürzt.

Der Einrichtung können sich ungeschulte Bedienungspersonen und sogar der Patient selbst ohne zusätzliche Anweisungen bedienen.

Durch die Verwendung der Einrichtung ist die Vereinigung der Einwirkung auf den Patienten und der Kontrolls seines Zustandes möglich.

Die Erfindung kann in der klinischen und Sportmedizin sowie als Gerät individueller Nutzung, insbesondere zur Prophylaxe und Behandlung von Krankheiten und pathologischen Zuständen mit gestörten Kompensations- und Adaptationsvorgangen verwendet werden.

## Ansprüche

1. Verfahren zur Erzeugung von für die Anregung biologischer Objekte bestimmten elektrischen Impulsen, bei dem man Impulse mit einer vorgegebenen Frequenz erzeugt, jeden Impuls längenmoduliert, Serien von Anregungsimpulsen mit einer vorgegebenen Seriendauer und einer vorgegebenen Pausenzeit zwischen ihnen unter linearer Verlängerung und Verkürzung der Dauer der Impulse vorgegebener Frequenz formiert, die eine Serie bildenden Anregungsimpulse verstärkt und diese über Elektroden einem biologischen Objekt zuführt, wobei man die maximale Dauer der Impulse vorgegebener Frequenz in der Serie je nach individueller Empfindlichkeit des biologischen Objektes einstellt, **dadurch gekennzeichnet,** daß man die Dauer der dem biologischen Objket zugeführten Anregungsimpulse entsprechend einer Auswertung elektrophysikalischer Zustandsparameter des Zwischenelektrodenbereiches des biologischen Objektes verändert, welche gleichzeitig mit der Anregung des biologischen Objektes durchgeführt wird.

2. Verfahren nach Anspruch 1, **dadurch gekennzeichnet,** daß als elektrophysikalischer Parameter zur Auswertung des Zustandes des biologischen Ovjektes die Impedanz des Zwischenelektrodenbereiches des biologischen Objektes benutzt wird.

3. Verfahren nach Anspruch 1 oder 2, **dadurch gekennzeichnet,** daß man die Impedanz des Zwischenelektrodenbereiches nach Zeitkennlinien eines elektrischen Signals bewertet, das nach der Beendigung jedes Anregungsimpulses entsent.

4. Verfahren nach Anspruch 1 oder 2, **dadurch gekennzeichnet,** daß die geänderte Dauer der Serie von Anregungsimpulsen mit der vorgegebenen Dauer verglichen wird, und wenn die geänderte Seriendauer die vorgegebene unterschreitet, die Zuführung der nächsten Anregungsimpulsserie zum betreffenden Bereich des biologischen Objektes wiederaufgenommen wird, und wenn die geänderte Dauer der Anregungsimpulsserie gleich der vorgegebenen oder länger als diese ist, die Zuführung der Anregungsimpulsserie zum betreffenden Bereich des biologischen Objektes abgebrochen wird.

5. Verfahren nach Anspruch 3, **dadurch gekennzeichnet**, daß die geänderte Dauer der Serie von Anregungsimpulsen mit der vorgegebenen Dauer verglichen wird, und wenn die geänderte Seriendauer die vorgegebene unterschreitet, die Zuführung der nächsten Anregungsimpulsserie zum betreffenden Bereich des biologischen Objektes wiederaufgenommen wird, und wenn die geänderte Dauer der Anregungsimpulsserie gleich der vorgegebenen oder länger als diese ist, die Zuführung der Anregungsimpulsserie zum betreffenden Bereich des biologischen Objektes abgebrochen wird.

6. Verfahren nach Anspruch 1 oder 2, **dadurch gekennzeichnet**, daß man invertierte elektrische Impulse zusätzlich formiert, mit denen auf den Bereich des biologischen Objektes eingewirkt wird.

7. Verfahren nach Anspruch 5, **dadurch gekennzeichnet**, daß man invertierte elektrische Impulse zusätzlich formiert, mit denen auf den Bereich des biologischen Objektes eingewirkt wird.

8. Einrichtung zur Erzeugung von für die Anregung biologischer Objekte bestimmten elektrischen Impulsen, die einen Impulsgenerator (6), einen Pulslängenmodulator (9), dessen einer Eingang am Ausgang des Impulsgenerators (6) liegt, einen Leistungsverstärker (10) für Anregungsimpulse, dessen Eingang mit dem Ausgang des Pulslängenmodulators (9) verbunden ist und dessen Ausgänge an die Elektroden (15, 16) angeschlossen sind, die an einen Bereich des biologischen Objekts bei dessen Anregung gelegt werden, enthält, **dadurch gekennzeichnet**, daß sie einen ersten Former (19) eines die Reaktion des biologischen Objektes auf den Anregungsimpuls bestimmenden Signals, dessen Eingang mit dem Ausgang des Angerungsimpuls-Leistungsverstärkers (10) verbunden ist und an dessen Ausgang ein Signal für die Steuerung der Dauer der Anregungsimpulsserie gebildet wird, einen Generator (22) für Impulse einer veränderbaren Dauer, die die Dauer der Anregungsimpulsserie festlegt und dessen Eingang mit dem Ausgang des ersten Signalformers (19) verbunden ist, einen zweiten Former (25) eines Signals, dessen Dauer der Dauer der Anregungsimpulsserie entspricht und das eine Vorder- und eine Hinterflanke aufweist, die sich mit einer konstanten Geschwindigkeit ändern, wobei der Former (25) eingangsseitig mit dem Ausgang des Generators (22) für Impulse einer veränderbaren Dauer verbunden ist, und einen Höchstpegelbegrenzer (26) für das Modulationssteuerungssignal enthält, dessen einer Eingang mit einer Signalpegelvorgabeschaltung (30), anderer Eingang mit dem Ausgang des zweiten Signalformers (25) und dessen Ausgang mit dem zweiten Eingang des Pulslängenmodulators (9) in Verbindung steht.

9. Einrichtung nach Anspruch 8, **dadurch gekennzeichnet**, daß sie einen Zeitdiskriminator (35) enthält, an dessen Ausgang ein die Beendigung der Anregung anzeigendes Signal gebildet wird und dessen einer Eingang mit dem Ausgang des Generators (22) für Impulse einer veränderbaren Dauer verbunden ist und dessen anderer Eingang am Ausgang einer Steuereinheit (39) des Zeitdiskriminators (35) liegt, die mit dem Ausgang des Anregungsimimpuls-Leistungsverstärkers (10) in Verbindung steht.

10. Einrichtung nach Anspruch 9, **dadurch gekennzeichnet**, daß der zweite Eingang der Steuereinheit (39) des Zeitdiskriminators (35) mit dem Ausgang des Impulsgenerators (6) verbunden ist.

11. Einrichtung nach Anspruch 9 oder 10, **dadurch gekennzeichnet**, daß der dritte Eingang des Zeitdiskriminators (35) mit dem Ausgang des Impulsgenerators (6) verbunden ist.

12. Einrichtung nach Anspruch 9 oder 10, **dadurch gekennzeichnet**, daß sie einen Zeitgeber (44), dessen Eingang mit dem Ausgang der Steuereinheit (39) des Zeitdiskriminators (35) verbunden ist, und einen steuerbaren Stromversorgungsteil (46) enthält, der eingangsseitig mit dem Ausgang des Zeitgebers (44) verbunden ist und die Stromversorgung abschaltet.

13. Einrichtung nach Anspruch 11, **dadurch gekennzeichnet**, daß sie einen Zeitgeber (44), dessen Eingang mit dem Ausgang der Steuereinheit (39) des Zeitdiskriminators (35) verbunden ist, und einen steuerbaren Stromversorgungteil (46) enthält, der eingangsseitig mit dem Ausgang des Zeitgebers (44) verbunden ist und die Stromversorgung abschältet.

14. Einrichtung nach Anspruch 11, **dadurch gekennzeichnet**, daß der zweite Eingang des Zeitgebers (44) mit dem Ausgang des Impulsgenerators (6) verbunden ist.

15. Einrichtung nach Anspruch 13, **dadurch gekennzeichnet**, daß der zweite Eingang des Zeitgebers (44) mit dem Ausgang des Impulsgenerators (6) verbunden ist.

16. Einrichtung nach Anspruch 9 oder 10, **dadurch gekennzeichnet**, daß der Ausgang des Zeitdiskriminators (35) mit dem dritten Eingang des Höchstpegelbegrenzers (26) für das Modulationssteuerungssignal verbunden ist.

17. Einrichtung nach Anspruch 13, **dadurch gekennzeichnet**, daß der Ausgang des Zeitdiskriminators (35) mit dem dritten Eingang des Höchstpegelbegrenzers (26) für das Modulationssteuerungssignal verbunden ist.

18. Einrichtung nach Anspruch 15, **dadurch gekennzeichnet**, daß der Ausgang des Zeitdiskriminators (35) mit dem dritten Eingang des Höchstpegelbegrenzers (26) für die Modulationssteuerungssignal verbunden ist.

19. Einrichtung nach Anspruch 12, **dadurch gekennzeichnet**, daß der Ausgang des Zeitdiskriminators (35) mit dem zweiten Eingang des steuerbaren Stromversorgungsteils (46) verbunden ist.

20. Einrichtung nach Anspruch 13, **dadurch gekennzeichnet**, daß der Ausgang des Zeitdiskriminators (35) mit dem zweiten Eingang des steuerbaren Stromversorgungsteils (46) in Verbindung steht.

21. Einrichtung nach Anspruch 15, **dadurch gekennzeichnet**, daß der Ausgang des Zeitdiskriminators (35) mit dem zweiten Eingang des steuerbaren Stromversorgunsteils (46) verbunden ist.

22. Einrichtung nach Anspruch 8 oder 9 oder 10 **dadurch gekennzeichnet**, daß sie einen Umschalter (52) der Elektroden (15, 16) aufweist, der zwischen den Elektroden (15, 16) und dem Anregungsimpuls-Leistungsverstärker (10) geschaltet ist.

23. Einrichtung nach Anspruch 15, **dadurch gekennzeichnet**, daß sie einen Umschalter (52) der Elektroden (15, 16) aufweist, der zwischen den Elektroden (15, 16) und dem Anregungsimpuls-Leistungsverstärker (10) geschaltet ist.

24. Einrichtung nach Anspruch 21, **dadurch gekennzeichnet**, daß sie einen Umschalter (52) der Elektroden (15, 16) aufweist, der zwischen den Elektroden (15, 16) und dem Anregungsimpuls-Leistungsverstärker (10) geschaltet ist.

25. Einrichtung nach Anspruch 8 oder 9 oder 10, **dadurch gekennzeichnet**, daß der erste Signalformer (19) und der Generator (22) für Impulse einer veränderbaren Dauer als Multivibrator mit CMOS-Transistoren (53 bis 56) ausgebildet sind, in dessen erster Stufe in den Sources jedes Transistors (53,54) Widerstände (59,60) geschaltet sind und an das Source des p-Kanal-Transistors (53) ein Kondensator (61) angeschlossen ist, dessen zweiter Anschluß den Eingang des ersten Signalformers (19) bildet, wobei die erste Stufe des Multivibrators einen Rückkopplungskreis aus einer Diode (63) und einem mit dieser in Reihe liegenden Widerstand (64) aufweist, und der Ausgang der zweiten Stufe des Multivibrators als Ausgang des Generators (22) für Impulse einer veränderbaren Dauer dient.

26. Einrichtung nach einem beliebigen der Anspruch 8 oder 9 oder 10 bis 15 **dadurch gekennzeichnet**, daß der zweite Signalformer (25) einen Integrator mit CMOS-Transistoren (66,67) darstellt, bei denen zwischen den Speiseschienen (68,69) und den Sources Widerstände (72,73) geschaltet sind.

27. Einrichtung nach Anspruch 25, **dadurch gekennzeichnet**, daß der zweite Signalformer (25) einen Integrator mit CMOS-Transistoren (66,67) darstellt, bei denen zwischen den Speiseschienen (68,69) und den Sources Widerstände (72,73) geschaltet sind.

28. Einrichtung nach Anspruch 8 oder 9 oder 10, **dadurch gekennzeichnet**, daß der Pulslängenmodulator (9) einen aus CMOS-Transistoren (74,75) aufgebauten Verstärker enthält, an dessen Eingang ein Differenzierkreis vorgesehen ist, dessen Zeitkonstante mit Hilfe eines einzelnen MOS-Transistors (79) geändert wird, dessen Gate mit einem Eingang des Pulslängenmodulators (9) verbunden ist, wobei der Eingang des Differenzierkreises als zweiter Eingang des Pulslängenmodulators (9) dient.

29. Einrichtung nach Anspruch 27, **dadurch gekennzeichnet**, daß der Pulslängenmodulator (9) einen aus CMOS-Transistoren (74,75) aufgebauten Verstärker enthält, an dessen Eingang ein Differenzierkreis vorgesehen ist, dessen Zeitkonstante mit Hilfe eines einzelnen MOS-Transistors (79) geändert wird, dessen Gate mit einem Eingang des Pulslängenmodulators (9) verbunden ist, wobei der Eingang des Differenzierkreises als zweiter Eingang des Pulslängenmodulators (9) dient.

30. Einrichtung nach Anspruch 8 oder 9 oder 10, **dadurch gekennzeichnet**, daß die Signalpegelvorgabeschaltung (30) einen Integrator mit CMOS-Transistoren (82,83) enthält, bei denen zwischen den Speiseschienen (84,85) und den Sources Widerstände (88,89) geschaltet sind, während der Eingang des Integrators über Umschalter (91,92) mit den Speiseschienen (84,85) in Verbindung steht.

31. Einrichtung nach Anspruch 29, **dadurch gekennzeichnet**, daß die Signalpegelvorgabeschaltung (30) einen Integrator mit CMOS-Transistoren (82,83) enthält, bei denen zwischen den Speiseschienen (84,85) und den Sources Widerstände (88,89) geschaltet sind, während der Eingang des Integrators über Umschalter (91,92) mit den Speiseschienen (84,85) in Verbindung steht.

32. Einrichtung nach Anspruch 8 oder 9 oder 10, **dadurch gekennzeichnet**, daß der Impulsgenerator (6) einen Multivibrator mit CMOS-Transistoren (93 bis 98) darstellt, dessen erste Stufe einen aus CMOS-Transistoren (95,96) aufgebauten Verstärker enthält, in dessen Rückkopplungskreis ein aus einem Widerstand (99) und einem Kondensator (100) bestehendes Trägheitsglied geschaltet ist, wobei zwischen den Speiseschienen (101,102) und den Sources der CMOS-Transistoren (95,96) der ersten Stufe noch je ein zusätzlicher MOS-Transistor (99 bzw.94) geschaltet ist und die zusammengeschalteten Gates der zusätzlichen MOS-Transistoren (93,94) den Eingang der ersten Stufe bilden, während der Ausgang des Verstärkers als Ausgang der ersten Stufe des Multivibrators dient.

33. Einrichtung nach Anspruch 31, **dadurch gekennzeichnet**, daß der Impulsgenerator (6) einen Multivibrator mit CMOS-Transistoren (93 bis 98) darstellt, dessen erste Stufe einen aus CMOS-Transistoren (95,96) aufgebauten Verstärker enthält, in dessen Rückkopplungskreis ein aus einem Widerstand (99) und einem Kondensator (100) bestehendes Trägheitsglied geschaltet ist, wobei zwischen den Speiseschienen (101,102) und den Sources der CMOS-Transistoren (95,96) der ersten Stufe noch je ein zusätzlicher MOS-Transistor (93 bzw. 94) geschaltet ist und die zusammengeschalteten Gates der zusätzlichen MOS-Transistoren (93,94) den Eingang der ersten Stufe bilden, während der Ausgang des Verstärkers als Ausgang der ersten Stufe des Multivibrators dient.

34. Einrichtung nach Anspruch 9 oder 10, **dadurch gekennzeichnet**, daß die Steuereinheit (39) des Zeitdiskriminators (35) - in Reihe geschaltet- einen Widerstand (109) und einen Negator (110), einen ersten Impulszähler (111), dessen Zähleingang mit dem Ausgang des Negators (110) verbunden ist und dessen Steuereingang an dessen Ausgang angeschlossen ist, der Rücksetzeingang dieses Impulszählers (111) als Eingang der Steuereinheit (39) dient, und einen zweiten Impulszähler (117) enthält, dessen Zähleingang mit dem Rücksetzeingang des ersten Impulszählers (111) vereinigt ist, wobei der Steuereingang des zweiten Impulszählers (117) mit seinem Ausgang und der Rücksetzeingang des zweiten Impulszählers (117) mit dem Ausgang des ersten Impulszählers (111) in Verbindung stehen.

35. Einrichtung nach Anspruch 9 oder 10, **dadurch gekennzeichnet**, daß der Zeitdiskriminator (35) ein ODER-Glied (122) und einen Impulszähler (123) enthält, dessen Rücksetzeingang mit dem Ausgang des ODER-Gliedes (122) verbunden ist, wobei der Zähleingang des Impulszählers (123) den Eingang des Zeitdiskriminators (35) bildet und der Steuereingang dieses Zählers (123) mit seinen Ausgang verbunden ist.

36. Einrichtung nach Anspruch 34, **dadurch gekennzeichnet**, daß der Zeitdiskriminator (35) ein ODER-Glied (122) und einen Impulszähler (123) enthält, dessen Rücksetzeingang mit dem Ausgang des ODER-Gliedes (122) verbunden ist, wobei der Zähleingang des Impulszählers (123) den Eingang des Zeitdiskriminators (35) bildet und der Steuereingang dieses Zählers (123) mit seinem Ausgang verbunden ist.

37. Einrichtung Anspruch 12, **dadurch gekennzeichnet**, daß der steuerbare Stromversorgungsteil (46) eine Spannungsquelle (129) und ein Flipflop (130) enthält, dessen Setzeingang über einen Ausschalter (132) mit dem Ausgang der Spannungsquelle (129) verbunden ist und dessen Rücksetzeingänge (130) als Eingänge des steuerbaren Stromversorgungsteils (46) dienen, wobei der Ausgang des Flipflops (130) mit dem Steuereingang eines elektronischen Schalters (138) verbunden ist, dessen Eingang an die Spannungsquelle (129) angeschlossen ist.

38. Einrichtung nach Anspruch 36, **dadurch gekennzeichnet**, daß der steuerbare Stromversorgungsteil (46) eine Spannungsquelle (129) und ein Flipflop (130) enthält, dessen Setzeingang über einen Ausschalter (132) mit dem Ausgang der Spannungsquelle (129) verbunden ist und dessen Rücksetzeingänge (130) als Eingänge des steuerbaren Stromversorgungsteils (46) dienen, wobei der Ausgang des Flipflops (130) mit dem Steuereingang eines elektronischen Schalters (138) verbunden ist, dessen Eingang an die Spannungsquelle (129) angeschlossen ist.

FIG. 1a

FIG. 1b

FIG. 1c

FIG. 1d

FIG. 1e

FIG. 2

FIG. 3

FIG. 4

FIG. 5

FIG. 6

FIG. 7

FIG. 8

FIG. 9

FIG.10

EP 0 425 673 A1

FIG. 11

FIG. 12

22

FIG. 14

FIG. 13

FIG. 15

FIG.16

FIG.17

FIG.18

FIG. 19

FIG. 20

FIG. 21

# INTERNATIONAL SEARCH REPORT

International Application No PCT/SU 89/00113

## I. CLASSIFICATION OF SUBJECT MATTER (If several classification symbols apply, indicate all) ⁶

According to International Patent Classification (IPC) or to both National Classification and IPC

Int.Cl⁵ ·· -     A 61 N 1/36 ··

## II. FIELDS SEARCHED

### Minimum Documentation Searched ⁷

| Classification System | Classification Symbols |
|---|---|
| Int.Cl.⁵ | A 61 N 1/32, 1/36, 1/362 |

Documentation Searched other than Minimum Documentation
to the Extent that such Documents are Included in the Fields Searched ⁸

## III. DOCUMENTS CONSIDERED TO BE RELEVANT⁹

| Category* | Citation of Document, ¹¹ with indication, where appropriate, of the relevant passages ¹² | Relevant to Claim No. ¹³ |
|---|---|---|
| A | US, A, 4733667 (Cardiac Pacemakers, Inc) 29 March 1988 (29.03.88), figure 1, the claims | 9 |
| A | US, A, 4669477 (Empi, Inc), 2 June 1987 (02.06.87), figures 1,2, page 3 | 1,8 |
| A | EP, B1, 0145176 (WACO CORPORATION OVERSEAS LIMITED et al.) 8 June 1988 (08.06.88) the claims, figure 5 | 8 |
| A | EP, A1, 0154976 (BIO-RESEARCH ASSOCIATES, Inc), 18 September 1985 (18.09.85), figures 2,6 | 25 |
| A | EP, A1, 0196393 (OSCOBALAG), 8 October 1986 (08.10.86), the claims, figures 3,4 | 8 |
| A | SU, A1, 1378849 (Moskovsky energetichesky institut), 7 March 1988 (07.03.88), the claims, the drawing | 2,3,8 |
| A | SU, A1, 865300 (Kuibyshevsky aviatsionny institut im. akademika S.P Koroleva) 25 September 1981 (25.09.81), the claims, figures 1,2 | 2,3,8 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 02 November 1989 (02.11.89) | 17 November 1989 (17.11.89) |

| International Searching Authority | Signature of Authorized Officer |
|---|---|
| ISA/SU | |

Form PCT/ISA/210 (second sheet) (January 1985)

| Category | Citation of Document, with indication, where appropriate, of the relevant passages | Relevant to Claim No |
|---|---|---|
| A | SU, A1, 921581 (Kuibyshevsky aviatsionny institut im. akademika S.P. Koroleva) 25 April 1982 (25.04.82), the claims, figures 1,2 | 2,6,8 |
| A | SU, A1, 1022715 (Moskovsky energetichesky institut), 15 June 1983 (15.06.83), the claims, the drawing | 2,3,8 |
| A | SU, A1, 1060192 (Kuibyshevsky aviatsionny institut im. akademika S.P.Koroleva), 15 December 1983 (15.12.83), the claims, the drawing | 2,6,8 |
| A | SU, A1, 1222282 (Kuibyshevsky aviatsionny institut im. akademika S.P.Koroleva) 7 April 1986 (07.04.86), the claims, figure 1 | 2,3,8 |
| A | SU, A1, 1242185 (Ju.P.Mironenko et al.), 7 July 1986 (07.07.86), claim 1, figure 1 | 2,6,8 |